(19) Europäisches Patentamt
European Patent Office
Office européen des brevets

(11) **EP 3 688 480 B1**

(12) **EUROPEAN PATENT SPECIFICATION**

(45) Date of publication and mention of the grant of the patent:
**29.05.2024 Bulletin 2024/22**

(21) Application number: **18779575.2**

(22) Date of filing: **18.09.2018**

(51) International Patent Classification (IPC):
**G01R 33/563** (2006.01)   **G01R 33/50** (2006.01)
**G01R 33/56** (2006.01)   **A61B 5/00** (2006.01)
**A61B 5/055** (2006.01)

(52) Cooperative Patent Classification (CPC):
**G01R 33/5608; A61B 5/004; A61B 5/055;**
**A61B 5/4381; G01R 33/50; G01R 33/56341;**
**G01R 33/56366;** A61B 5/0037

(86) International application number:
**PCT/EP2018/075117**

(87) International publication number:
**WO 2019/063342 (04.04.2019 Gazette 2019/14)**

(54) **NON-INVASIVE ESTIMATION OF PROSTATE TISSUE COMPOSITION BASED ON MULTI-PARAMETRIC MRI DATA**

NICHT-INVASIVE SCHÄTZUNG DER ZUSAMMENSETZUNG DES PROSTATAGEWEBES BASIEREND AUF MULTIPARAMETRISCHEN MRT-DATEN

ESTIMATION NON INVASIVE DE COMPOSITION DE TISSU DE PROSTATE SUR LA BASE DE DONNÉES D'IRM MULTIPARAMÉTRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.09.2017 US 201762563362 P**
**28.06.2018 US 201862691268 P**

(43) Date of publication of application:
**05.08.2020 Bulletin 2020/32**

(73) Proprietors:
• **Koninklijke Philips N.V.**
  **5656 AG Eindhoven (NL)**
• **The University of Chicago**
  **Chicago, IL 60637 (US)**

(72) Inventors:
• **DEVARAJ, Ajit**
  **5656 AE Eindhoven (NL)**
• **KARCZMAR, Gregory Stanislaus**
  **5656 AE Eindhoven (NL)**
• **OTO, Aytekin**
  **5656 AE Eindhoven (NL)**
• **CHATTERJEE, Aritrick**
  **5656 AE Eindhoven (NL)**

(74) Representative: **Philips Intellectual Property & Standards**
**High Tech Campus 52**
**5656 AG Eindhoven (NL)**

(56) References cited:
• **JEROME N P ET AL: "Extended T2-IVIM model for correction of TE dependence of pseudo-diffusion volume fraction in clinical diffusion-weighted magnetic resonance imaging", PHYSICS IN MEDICINE AND BIOLOGY, INSTITUTE OF PHYSICS PUBLISHING, BRISTOL GB, vol. 61, no. 24, 28 November 2016 (2016-11-28), XP020311566, ISSN: 0031-9155, DOI: 10.1088/1361-6560/61/24/N667 [retrieved on 2016-11-28]**
• **IVAN JAMBOR ET AL: "Optimization of b-value distribution for biexponential diffusion-weighted MR imaging of normal prostate", JOURNAL OF MAGNETIC RESONANCE IMAGING, vol. 39, no. 5, 11 October 2013 (2013-10-11), pages 1213-1222, XP055530593, US ISSN: 1053-1807, DOI: 10.1002/jmri.24271**

**(Cont. next page)**

- DEANNA L LANGER ET AL: "Prostate Tissue Composition and MR Measurements: Investigating the Relationships between ADC, T2, Ktrans, ve, and Corresponding Histologic Features", RADIOLOGY, vol. 255, no. 2, 8 April 2010 (2010-04-08), pages 485-494, XP055529502, US ISSN: 0033-8419, DOI: 10.1148/radiol.10091343 cited in the application
- ANNA S. RYDHÖG ET AL: "Separating blood and water: Perfusion and free water elimination from diffusion MRI in the human brain", NEUROIMAGE, vol. 156, 13 April 2017 (2017-04-13), pages 423-434, XP055530617, AMSTERDAM, NL ISSN: 1053-8119, DOI: 10.1016/j.neuroimage.2017.04.023
- YOUSEF MAZAHERI ET AL: "Prostate Tumor Volume Measurement with Combined T2-weighted Imaging and Diffusion-weighted MR: Correlation with Pathologic Tumor Volume", RADIOLOGY, vol. 252, no. 2, 1 August 2009 (2009-08-01), pages 449-457, XP055529609, US ISSN: 0033-8419, DOI: 10.1148/radiol.2523081423
- ROGER M. BOURNE ET AL: "Apparatus for Histological Validation of In Vivo and Ex Vivo Magnetic Resonance Imaging of the Human Prostate", FRONTIERS IN ONCOLOGY, vol. 7, 47, 24 March 2017 (2017-03-24), pages 1-8, XP055530449, DOI: 10.3389/fonc.2017.00047
- JÖRG DÖPFERT ET AL: "Investigation of prostate cancer using diffusion-weighted intravoxel incoherent motion imaging", MAGNETIC RESONANCE IMAGING, ELSEVIER SCIENCE, TARRYTOWN, NY, US, vol. 29, no. 8, 19 August 2011 (2011-08-19), pages 1053-1058, XP028287144, ISSN: 0730-725X, DOI: 10.1016/J.MRI.2011.06.001 [retrieved on 2011-07-15]
- MELBOURNE ANDREW ET AL: "Multi-modal Measurement of the Myelin-to-Axon Diameter g-ratio in Preterm-born Neonates and Adult Controls", 14 September 2014 (2014-09-14), SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER], SPRINGER, BERLIN, HEIDELBERG, PAGE(S) 268 - 275, XP047318877, ISBN: 978-3-540-74549-5
- ANNA SCHERMAN RYDHÖG ET AL: "Multidimensional Diffusion and Relaxation Data Acquisition for Improved Intravoxel Incoherent Motion Analysis", PROCEEDINGS OF THE INTERNATIONAL SOCIETY FOR MAGNETIC RESONANCE IN MEDICINE, 24TH ANNUAL MEETING AND EXHIBITION, SINGAPORE, 7-13 MAY 2016, vol. 24, 1897, 22 April 2016 (2016-04-22), XP040682939,

## Description

### FIELD

The following relates generally to medical imaging arts, glandular tissue monitoring arts, non-invasive tissue monitoring arts, oncology and oncological imaging arts, using multi-parametric magnetic resonance imaging.

### BACKGROUND

Prostate cancer is the most common non-cutaneous cancer and second leading cause of death among men in the United States (see, e.g., Siegel R, Naishadham D, Jemal A. Cancer statistics, 2012. CA: A Cancer Journal for Clinicians. 2012;62(1):10-29; and Johnson LM, Choyke PL, Figg WD, Turkbey B. The Role of MRI in Prostate Cancer Active Surveillance. BioMed Research International. 2014;2014:6.). A key to effective screening and treatment planning is the knowledge of lesion aggressiveness. The currently accepted approach to assessing the aggressiveness of prostate cancer is the Gleason grading system which is based on tissue architectural changes evaluated on histology. The tissue architectural changes described by the Gleason grading system are directly related to the prostate tissue microstructure i.e. tissue composition (see, e.g., Chatterjee A, Watson G, Myint E, Sved P, McEntee M, Bourne R. Changes in Epithelium, Stroma, and Lumen Space Correlate More Strongly with Gleason Pattern and Are Stronger Predictors of Prostate ADC Changes than Cellularity Metrics. Radiology. 2015;277(3):751-62; Langer DL, van der Kwast TH, Evans AJ, et al. Prostate tissue composition and MR measurements: investigating the relationships between ADC, T2, K(trans), v(e), and corresponding histologic features. Radiology. 2010;255(2):485-94.; Kobus T, Laak JAWMvd, Maas MC, et al. Contribution of Histopathologic Tissue Composition to Quantitative MR Spectroscopy and Diffusion-weighted Imaging of the Prostate. Radiology. 2015;278(3):801-11; and Zhao M, Myint E, Watson G, Bourne R. Comparison of conventional histology and diffusion weighted microimaging for estimation of epithelial, stromal, and acinar volumes in prostate tissue. In Proceedings of the 21st Annual Meeting of ISMRM. Salt Lake City, Utah, USA2013; p. 3090).

Magnetic Resonance Imaging (MRI) microscopy of fixed prostate tissue using a preclinical 16T MR has been used to image individual prostatic glands at sufficient resolution (40$\mu$m isotropic) and demonstrated the different MR properties of gland microstructures and changes in these microstructures associated with cancer (see, e.g., Bourne RM, Kurniawan N, Cowin G, et al. Microscopic diffusivity compartmentation in formalin-fixed prostate tissue. Magn Reson Med. 2012;68(2):614-20; and Bourne R, Kurniawan N, Cowin G, Sved P, Watson G. 16 T diffusion microimaging of fixed prostate tissue: preliminary findings. Magn Reson Med. 2011;66(1):244-7). However, the spatial resolution of clinical MR scans is far too low to resolve glandular structures as prostate acini are approximately 0.1 mm in diameter (see, e.g., Bourne R. Magnetic resonance microscopy of prostate tissue: How basic science can inform clinical imaging development. Journal of Medical Radiation Sciences. 2013;60(1):5-10).

Multi-parametric magnetic resonance imaging (mpMRI) is increasingly used in the detection, characterization, and staging of prostate cancer. Most of the current MR biomarkers (e.g. ADC and T2W images) are based on phenomenological models: mono-exponentials, bi-exponentials and kurtosis models. While these phenomenological model based markers are widely accepted for prostate cancer detection clinically, a large number of prostate cancers go undetected, especially smaller significant lesions often go undetected, due to their similarity to the normal gland and benign conditions (see, e.g., Kitzing YX, Prando A, Varol C, Karczmar GS, Maclean F, Oto A. Benign Conditions That Mimic Prostate Carcinoma: MR Imaging Features with Histopathologic Correlation. RadioGraphics. 2016;36(1):162-75; and Rosenkrantz AB, Taneja SS. Radiologist, Be Aware: Ten Pitfalls That Confound the Interpretation of Multiparametric Prostate MRI. American Journal of Roentgenology. 2013;202(1):109-20) and they provide limited information to characterise the lesions. This usually leaves the radiologist with just the legion size and number and maybe longitudinal tracking information to assess cancer aggressiveness. Therefore histological changes remain the reference standard for evaluating cancer aggressiveness.

Studies based on structural models like the 'vascular, extracellular, and restricted diffusion for cytometry in tumors' (VERDICT) (see, e.g., Panagiotaki E, Chan RW, Dikaios N, et al. Microstructural Characterization of Normal and Malignant Human Prostate Tissue With Vascular, Extracellular, and Restricted Diffusion for Cytometry in Tumours Magnetic Resonance Imaging. Investigative radiology. 2015;50(4):218-27) characterize diffusion in three distinct microstructural compartments and may help characterize prostate cancer with good accuracy. However, this model uses increased cellularity as the primary marker for cancer. This is significantly different from histological markers that are known to correlate with prostate cancer (see, e.g., Selnæs KM, Vettukattil R, Bertilsson H, et al. Tissue Microstructure Is Linked to MRI Parameters and Metabolite Levels in Prostate Cancer. Frontiers in Oncology. 2016;6(146)).

A recent study showed that fractional volumes of the prostate gland components: stroma, epithelium, and lumen correlate more strongly with both cancer presence and Gleason grade than 'cellularity' metrics. There is an increase epithelial volume with a reduction in luminal and stromal volume in cancers. These changes are more pronounced with increasing Gleason grade. In addition, the tissue components: stroma, epithelium and lumen have distinct diffusivities, and these diffusivities therefore might be used as biomarkers for non-invasive prostate cancer detection. Two recent studies showed that luminal volume estimated using a bi-exponential model to fit multi-echo T2-weighted images, or Luminal Water Imaging (LWI) could be used for detection of prostate cancer (see, e.g., Sabouri S, Fazli L, Chang SD, et al. MR measurement of luminal water in prostate gland: Quantitative correlation between MRI and histology. Journal of Magnetic Resonance Imaging. 2017:n/a-n/a; and Sabouri S, Chang SD, Savdie R, et al. Luminal Water Imaging: A New MR Imaging T2 Mapping Technique for Prostate Cancer Diagnosis. Radiology. 2017;0(0): 161687).

The following discloses new and improved systems and methods to overcome these problems.

A document entitled "Extended T2-IVIM model for correction of TE dependence of pseudo-diffusion volume fraction in clinical diffusion-weighted magnetic resonance imaging", by Jerome, N. P. et al., Phys. Med. Biol. 61 (2016) N667-N680, discloses an extended model, T2-IVIM, that allows removal of the confounding echo-time (TE) dependence of *f* and provides direct compartment T2 estimates in relation to a bi-exponential intravoxel-incoherent-motion (IVIM) model for diffusion-weighted MRI (DWI).

A document by Melbourne, A., et al., "Multi-modal Measurement of the Myelin-to-Axon Diameter g-ratio in Preterm-born Neonates and Adult Controls", September 2014, SAT 2015 18th International Conference; Lecture Notes in Computer Science, Pages 268-275, discloses that infants born prematurely are at increased risk of adverse functional outcome. The measurement of white matter tissue composition and structure can help predict functional performance and this motivates the search for new multi-modal imaging biomarkers. In this work, a novel combined biomarker is developed from diffusion MRI and multi-component T2 relaxation measurements in a group of infants born very preterm and scanned between 30 and 40 weeks equivalent gestational age. This biomarker is investigated on a group of seven adult controls, using a multi-modal joint model-fitting strategy. The proposed emergent biomarker is tentatively related to axonal energetic efficiency (in terms of axonal membrane charge storage) and conduction velocity and is thus linked to the tissue electrical properties, giving it a good theoretical justification as a predictive measurement of functional outcome.

Another document by Rydhög, A. S., et al., "Multidimensional Diffusion and Relaxation Data Acquisition for Improved Intravoxel Incoherent Motion Analysis", Proceedings of the International Society for Magnetic Resonance in Medicine, Vol. 24, 1897, April 2016, discloses that Intravoxel Incoherent Motion "IVIM" is a method for quantification of perfusion parameters, such as the perfusion fraction Fb. Unfortunately, CSF partial volume effects are often seen in the estimated blood compartment. This work introduces a novel version of the IVIM model, containing three compartments (tissue, CSF and blood), where multi-TE and multi-TI data are incorporated to yield a direct relaxation estimate. Using this relaxation-compensated model, results were obtained from in vivo measurements in a volunteer. Compared to a non-relaxation-compensated model, the three-compartment model with relaxation-compensated data reduced the CSF contamination.

## SUMMARY

In one disclosed aspect, in accordance with independent claim 1, a non-transitory storage medium stores instructions readable and executable by at least one electronic processor , wherein the instructions when executed by the at least one electronic processor cause the at least one electronic processor to perform an imaging method The imaging method includes: obtaining multi-parametric magnetic resonance (MR) imaging data parameterized by a diffusion weighting or perfusion weighting parameter and a magnetization relaxation parameter for a gland of a patient; determining volume fraction maps of the gland for each of a plurality of gland tissue compartments from the multi-parametric MR imaging data; the gland tissue compartments comprising a stromal compartment, an epithelial compartment, and a lumen compartment; and controlling a display device to display a tissue composition map comprising or generated from the determined volume fraction maps.

In another disclosed aspect, in accordance with independent claim 15, a medical imaging workstation includes a processor configured to generate a prostatic tissue composition map (pTCM) by transforming a multi-dimensional array of multi-parametric magnetic resonance (MR) imaging data parameterized by a diffusion weighting or perfusion weighting parameter and a magnetization relaxation parameter for a prostate of a patient using a model to non-invasively evaluate maps of volume fractions, ADC's, and T2's of prostate tissue compartments; wherein the processor is configured to obtain the multi-parametric MR imaging data; and wherein the prostate tissue compartments comprise a stromal compartment, an epithelial compartment, and a lumen compartment.

One advantage resides in non-invasively detecting potential tumors in a target organ of a patient.

Another advantage resides in providing for non-invasive grading of cancer.

Another advantage resides in detecting potential tumors in the target organ without obtaining a biopsy.

Another advantage resides in providing for simultaneous imaging of the volume fractions of a plurality of different tissue types.

Another advantage resides in measuring properties of each compartment based on the ADC and T2 of each compartment.

Another advantage resides in detecting and measuring an additional compartment of water with a restricted diffusion and a long T2 value that is associated with cancer.

A given embodiment may provide none, one, two, more, or all of the foregoing advantages, and/or may provide other advantages as will become apparent to one of ordinary skill in the art upon reading and understanding the present disclosure.

## BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.

FIGURE 1 diagrammatically shows an illustrative imaging system in accordance with one aspect;
FIGURE 2 shows an exemplary flow chart operation of the system of FIGURE 1;
FIGURE 3 shows an example schematic of a map generated by the system of FIGURE 1; and
FIGURES 4 and 5 show results of an imaging method performed by the system of FIGURE 1.

## DETAILED DESCRIPTION

MRI prostate cancer imaging can be used preparatory to surgery (i.e. MRI planning images), or for screening, guiding biopsies, cancer grading, non-invasive monitoring, or so forth. T2 imaging or diffusion weighted imaging (DWI) are common imaging techniques for this purpose. However, these techniques have certain deficiencies, due to interrelatedness of the T2 and DWI contrast mechanisms.

In embodiments disclosed herein, a parameterized model of the MRI signal is developed, which defines various tissue types (stroma, epithelium, and lumen in the case of the prostate or some other glands; the tissue types are sometimes referred to as tissue compartments). The model parameters include volume fraction, T2, and apparent diffusion coefficient (ADC) for each tissue type. For the stroma/epithelium/lumen model, this results in nine model parameters. By acquiring multi-parametric magnetic resonance (MR) imaging data, such as, for example, a 3x3 matrix of different (TE,b-value) pairs (or, more generally, 9 different (TE,b-value) pairs), nine equations can be created by applying the model to predict the MR signal for each (TE,b-value) pair. Additionally, the partial volumes must sum to unity, providing a tenth equation. Thus, for each voxel there are nine unknowns and ten equations. This model applied to the 3x3 multi-parametric MR imaging data can therefore be solved for the volume fractions of the stroma, epithelium, and lumen at each voxel. The resulting volume fraction values for each voxel may be displayed as a prostatic tissue composition map (pTCM). This may be displayed as three maps (one for each tissue type) or as a composite map, e.g. using color coding. Larger matrices have also been used, but this increases acquisition time.

These techniques can be generalized to other parameter pairs (magnetization relaxation parameter, DWI parameter besides (TE,b-value)). For example, b-value adjustment can be obtained by varying the diffusion gradient amplitude or the diffusion time between gradients. The magnetization relaxation parameter may for example be the echo time (TE),the flip angle, the time-to-repeat (TR), pulses that induce magnetization transfer, or other parameters. Additionally, in some embodiments the DWI parameterization is replaced by Diffusion Contrast Enhanced (DCE) imaging parameterization, i.e. the multi-parametric MR imaging data may be parameterized using (magnetization relaxation parameter, DCE parameter) pairs.

The disclosed systems and methods can be applied to any gland-based organ having stroma, epithelium, and lumen tissue types (for example, prostate, colorectal, breast, pancreas, or thyroid)

In embodiments disclosed herein, all nine parameters obtained from a three compartment model are solved independently at each voxel. In variant envisioned approaches, various correlations may be leveraged as constraints or additional limitations, such as a constraint that the T2 and/or ADC parameters are slowly varying (or even constant) over the volume. Additionally, it is contemplated to acquire more than 9 different (TE,b-value) combinations, and/or for those combinations to not form a 3x3 matrix.

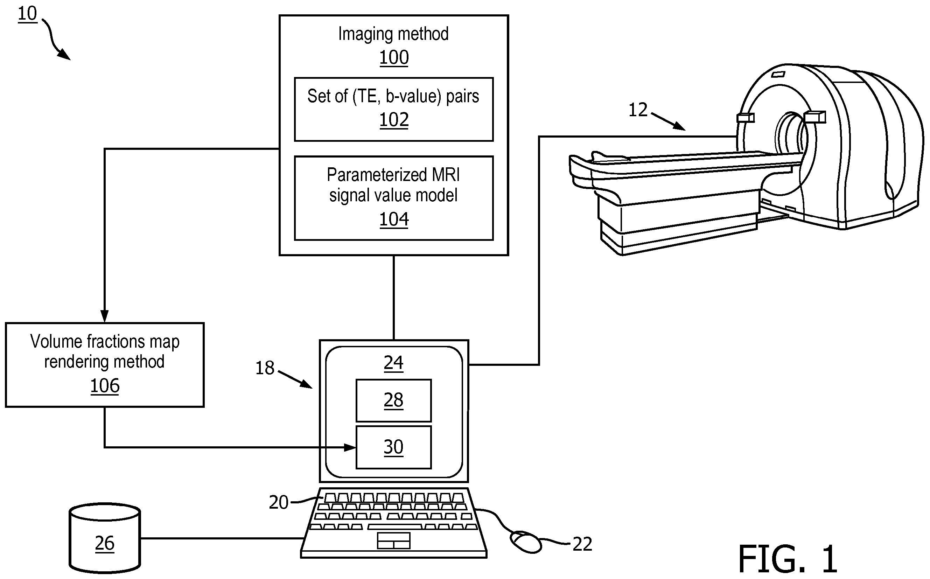

With reference to FIGURE 1, an imaging system or device **10** is shown. As shown in FIGURE 1, the device **10** includes a magnetic resonance (MR) imaging device **12** configured to acquire MR imaging data using a DWI sequence with diffusion gradients designed for a particular b-value (or, in alternative embodiment, using a DCE which can be used to produce parameters such as Ktrans (computed from the images as an imaging biomarker), ve, maximum rate of enhancement, and time of initial enhancement.

The device **10** also includes a medical imaging workstation **18** comprising a computer or other electronic data processing device with typical components, such as at least one electronic processor **20,** at least one user input device (e.g., a mouse, a keyboard, a trackball, and/or the like) **22,** and a display device **24.** It should be noted that these components can be variously distributed. For example, the electronic processor **20** may include a local processor of a workstation terminal and the processor of a server computer that is accessed by the workstation terminal. In some embodiments, the display device **24** can be a separate component from the computer **18.** The workstation **18** can also include one or more databases or non-transitory storage media **26.** The non-transitory storage media **26** may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth. The display device **24** is configured to display a graphical user interface (GUI) **28** including one or more fields to receive a user input from the user input device **22.** A generated map **30** of the volume fractions of the various tissue types (described in more detail below) can be displayed on the display device **24.**

The system **10** comprises the medical imaging workstation **18** with the processor **20** configured to generate from an MRI image a prostatic tissue composition map (pTCM) by transforming a multi-dimensional array of MRI data using a model to non-invasively evaluate volume fractions, ADC's, and T2's of prostate tissue components. In illustrative examples, the tissue components comprise stroma, epithelium and lumen which is a suitable tissue compartmentalization model for the prostate and for some other glands or glandular tissue structures such as the rectum and/or colon, breast, pancreas, or thyroid gland.

The system **10** is configured to perform an imaging method or process **100** which acquires multi-parametric MR imaging data for a set of (magnetization relaxation parameter, DWI or DCE parameter) pairs **102,** and more particularly for a set of (TE,b-value) pairs **102** in the example for prostate cancer imaging of FIGURE 1, and applies a parameterized MRI signal value model **104** to the acquired multi-parametric MR imaging data to generate volume fraction values for the various tissue types of the model **104** (according to the invention, for the stroma, epithelial, and lumen tissue types as in the illustrative prostate cancer imaging case) for each voxel of a region of interest (ROI), thereby forming volume fraction maps for the various tissue types. A volume fractions map rendering method **106** generates a one or more map renderings, i.e. map(s) **30,** of the volume fractions mapping data output by the imaging method or process **100,** which is or are suitably displayed on the display **24.** A non-transitory storage medium (e.g., non-transitory storage media **26)** stores instructions which are readable and executable by the at least one electronic processor **20** of the workstation **18** and to perform disclosed operations including performing the treatment planning method or process **100** and the volume fractions map rendering method **106.** In some examples, one or both methods **100, 106** may be performed at least in part by cloud processing.

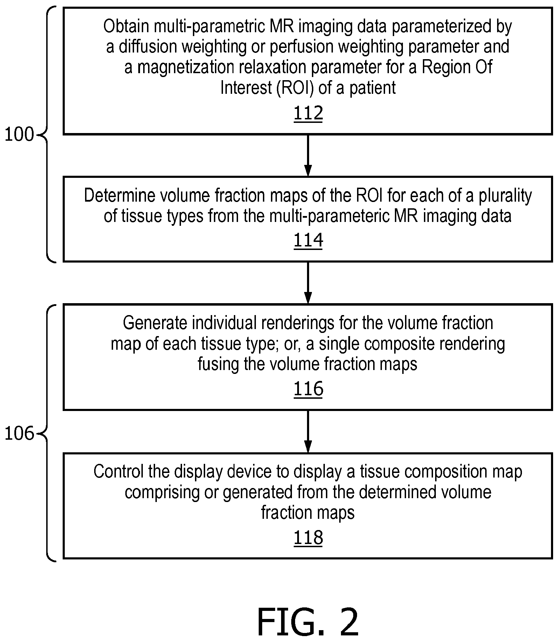

With reference to FIGURE 2, an illustrative embodiment of the treatment planning method **100** and the rendering method **106** is diagrammatically shown as a flowchart. At **112,** the at least one electronic processor **20** is programmed to acquire multi-parametric MR imaging data parameterized by a diffusion weighting or perfusion weighting parameter values and different magnetization relaxation parameter values (that is, for the set of parameter value pairs **102,** see FIGURE 1) for a region of interest (ROI) of a patient. In some examples, the at least one of a diffusion weighting or perfusion parameter includes a diffusion weighting parameter, and the diffusion weight parameter comprises one of a b-value, a diffusion time, or a diffusion gradient. In other examples, the at least one of a diffusion weighting or perfusion parameter value includes a perfusion weighting parameter. In further examples, the at least one magnetization relaxation parameter comprises one of echo time ($T_E$) or flip angle. In some examples embodiments, the at least one magnetization relaxation parameter value comprises echo time ($T_E$) and the diffusion weighting parameter value comprises a b-value.

At **114,** the at least one electronic processor **20** is programmed to determine volume fraction maps of the ROI for each of a plurality of tissue types from the multi-parametric MR imaging data using the parameterized MRI signal value model **104** (see FIGURE 1). According to the invention, the plurality of tissue types includes a stroma tissue type, an epithelial tissue type, and a lumen tissue type. In some examples, the determining includes solving a system of equations for each voxel to determine the volume fraction values for each tissue type of the plurality of tissue types, where the system of equations for each voxel includes an equation for each value pair of: (i) the diffusion weighting or perfusion weighting parameter, and the (ii) magnetization relaxation parameter, in the multi-parametric MR imaging data. In addition, the system of equations for each voxel further includes an equation requiring that the sum of the volume fractions equals one. Other constraints or limitations may optionally be incorporated. By this process, the volume fractions along with any other variables of the model **104** (e.g., the $T_2$ values and apparent diffusion coefficient (ADC) values in the illustrative example) are determined for each tissue type of the plurality of tissue types.

In some embodiments, the multi-parametric MR imaging data includes MR imaging data acquired for at least nine ($T_E$,b-value) parameter value pairs. To determine the volume fraction maps, the determining of the volume fraction maps includes solving a system of equations at each voxel for at least the volume fraction of each tissue type. The system of equations includes, for each ($T_E$,b-value) parameter value pair, an equation relating MR signal of the voxel acquired with the ($T_E$,b-value) parameter value pair to a weighted sum of signal components for each tissue type functionally depending on relaxation time $T_2$ and ADC of the tissue type with each signal component weighted by the volume fraction of the tissue type. The equation can also be expressed as solving equations at each voxel for at least the volume fraction $V_{Ti}$ of each tissue type $T_i \in \{T\}$ where $\{T\}$ denotes the set of the plurality of tissue types, wherein the system of equations includes, for each ($T_E$,b-value) parameter value pair, The equation can be expressed as Equation 1:

$$\frac{S}{S_0} = \sum_{T_i \in \{T\}} V_{T_i} \exp\left(-\frac{T_E}{T_{2_{T_i}}}\right) \exp\left(-(b \times ADC_{T_i})\right) \qquad (1)$$

where $S$ is the MR signal at the voxel, $S_0$ is a constant, $T_E$ and b are the echo time $T_E$ and b-value, respectively, of the $(T_E, \text{b-value})$ parameter value pair and $T_{2_{T_i}}$ and $ADC_{Ti}$ denote the relaxation time $T_2$ and ADC of the tissue type $T_i$. Thus, as demonstrated in Equation 1, the system of equations at each voxel is solved for the volume fraction of each tissue type and relaxation time $T_2$ and ADC of each tissue type (e.g., $T_{2_{T_i}}$ and $ADC_{Ti}$).

Furthermore, the system of equations at each voxel further includes an equation requiring that the sum of the volume fractions equals one. This can be expressed as Equation 2:

$$\sum_{T_i \in \{T\}} V_{T_i} = 1 \qquad (2)$$

requiring that the sum of the volume fractions equals one. Additional or other equations and/or constraints are contemplated to provide a more overdetermined and/or constrained set of equations. For example, the $T_2$ and/or ADC values may be constrained to a physically realistic range, using either hard constraints (e.g., not allowing the fitted $T_2$ to go outside of some specified physically realistic range) or soft constraints (e.g., penalizing $T_2$ by an increasingly large penalty value the further the fitted $T_2$ strays from a prior-known "typical" value of $T_2$ for the tissue). Another contemplated constraint is to penalize excessive spatial variation - this approach can improve smoothness of the fit, albeit at a cost of greater computational complexity due to the equations for neighboring voxels being interdependent. As another contemplated approach, $T_2$ and/or ADC for a particular tissue may be set to a constant "typical" value for the tissue, which simplifies computational complexity and may smooth the data, but at a possible cost of some increased error in the determined volume fractions.

At **116,** the at least one electronic processor **20** is programmed to generate individual renderings for the volume fraction map of each tissue type. This can be done, for example, by rendering each volume fraction map (one for stroma, one for epithelium, and one for lumen, in the illustrative prostate example) as a grayscale or color-coded rendering of the volume fraction values of the voxels of the ROI. Alternatively, operation **116** may generate a single composite rendering that fuses the volume fraction maps. One approach for this is to employ a red-green-blue (RGB) color model (or some other chosen three-component color model) and to set the intensity of each color component of each voxel of the rendering to the voxel volume fraction of a corresponding tissue type. Optionally, both individual tissue type renderings and a single composite rendering may be generated at operation **116.**

At **118,** the at least one electronic processor **20** is programmed to control the display device **24** to display the rendering(s) generated at operation **116,** e.g. to display a tissue composition map comprising or generated from the determined volume fraction maps. In one example, the display device **24** is controlled to display the tissue composition map as a display of the volume fraction map for each tissue type of the plurality of tissue types. In another example, the display device **24** is controlled to display the tissue composition map as a single composition map fusing the volume fraction maps for the plurality of tissue types.

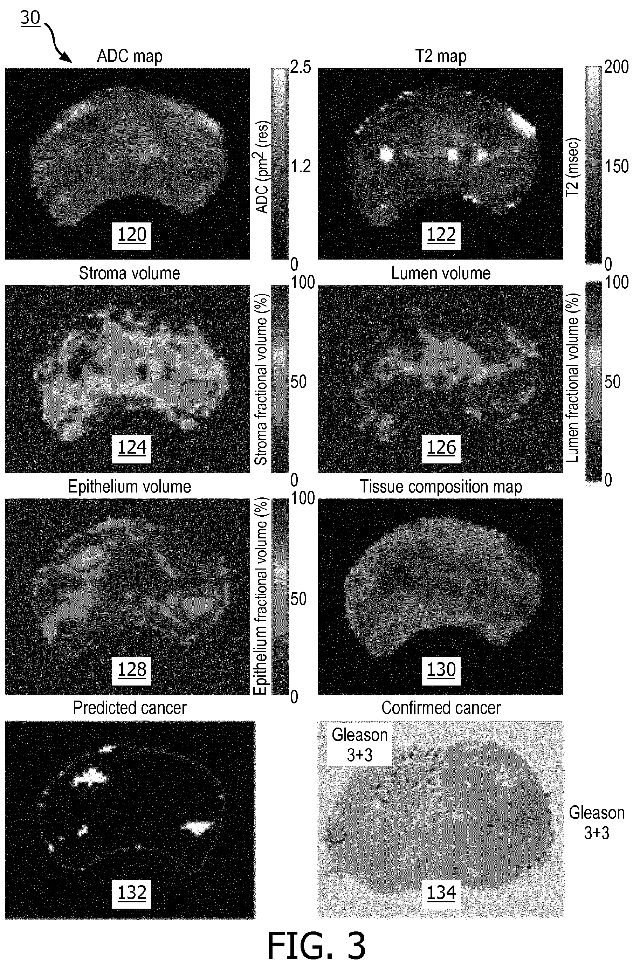

FIGURE 3 shows several examples of composite maps **30** for display on the display device **24**. The maps **30** shown in FIGURE 3 include: the ADC map **120** and the $T_2$ map **122;** the individual volume fraction maps, namely the stroma volume fraction map **124,** the lumen volume fraction map **126,** and the epithelium volume fraction map **128;** a prostatic tissue composition map (pTCM) **130;** and a predicted cancer map **132** derived from the volume fraction maps **124, 126, 128** and a corresponding map **134** showing confirmed cancer on, for example, hematoxylin and eosin stained tissue.

EXAMPLE

Advantageously, the disclosed method **100** can be validated against quantitative histology. Specifically, MRI provides measurements of epithelial, stromal, and lumen volume fractions that closely agree with quantitative histology. This demonstrates for the first time the use of *in vivo* MRI to measure volume fractions of epithelia, stroma, and lumen non-invasively (see below and in Chatterjee, A., Bourne, R.M., Karczmar, G.S., Oto, A. et al, Diagnosis of Prostate Cancer with Noninvasive Estimation of Prostate Tissue Composition by using Hybrid Multidimensional MR imaging: A Feasibility Study., Radiology, 2018).

If it is assumed that ADC and T2 are independent parameters, then components with different ADC's may be identified from modelling diffusion data and components with different T2's may be identified from spin echo measurements (see, e.g., Storås TH, Gjesdal K-I, Gadmar ØB, Geitung JT, Kløw N-E. Prostate magnetic resonance imaging: Multiexponential T2 decay in prostate tissue. Journal of Magnetic Resonance Imaging. 2008;28(5):1166-72; and Kjaer L, Thomsen C, Iversen P, Henriksen O. In vivo estimation of relaxation processes in benign hyperplasia and carcinoma of the prostate gland by magnetic resonance imaging. Magnetic resonance imaging. 1987;5(1):23-30). However, in this approach it is not known whether the different components identified by diffusion and T2 measurements have a direct one-to-one correspondence (see, e.g., Bourne R, Panagiotaki E. Limitations and Prospects for Diffusion-Weighted MRI of the Prostate. Diagnostics. 2016;6(2):21, and Wange, S., Oto, A., Karczmar, G.S. eta I, Hybrid Multidimentional T2 and diffusion-weighted MRI for prostate cancer detection, J Magnetic Resonance Imaging, 2014).

For example, by co-registering *in vivo* MRI with Hematoxylin and Eosin stained whole mount prostatic tissue, - the volume fractions of epithelium, stroma, and lumens measured by *in vivo* HM-MRI agree closely with gold standard histology. Specifically, patients ($n$ = 8, age = 62 years, PSA = 15.0 ng/ml) with biopsy confirmed PCa underwent preoperative 3T prostate MRI prior to undergoing radical prostatectomy. Axial images using HM-MRI were acquired with all combinations of TE = 57, 70, 150, 200 ms and *b*-values of 0, 150, 750, 1500 s/mm$^2$, resulting in a 4×4 array of data associated with each voxel. Volumes of tissue components- stroma, epithelium and lumen were calculated by fitting the hybrid data to a three compartment signal model, with distinct, paired ADC and T2 values associated with each compartment. Quantitative histology was performed to calculate volumes of tissue components on rectangular ROIs from region corresponding to the MR ROIs ($n$=16, 8 PCa, 8 normal tissue) using Image Pro Premier.

There was no significant difference (paired t-test) in prostate tissue composition: stroma (43.8±11.8 vs 41.6±8.7, *p*=0.20), epithelium (34.2±18.1 vs 36.7±15.3, *p*=0.07) and lumen (22.0±13.1 vs 21.7±10.9, *p*=0.80) measured using HM-MRI and quantitative histology. There was excellent Pearson correlation (overall = 0.94, stroma = 0.84, epithelium = 0.97, lumen = 0.93, *p* < 0.05) between prostate tissue composition measured using these methods. ROC analysis showed high area under the curve using HM-MRI (epithelium = 0.89, lumen = 1.00) and histology (epithelium = 0.91, lumen = 0.93) results, suggesting PCa can be differentiated from normal tissue estimated non-invasively by HM-MRI due to increased epithelium and reduced luminal volume in PCa compared to normal tissue.

For example, when ADC and T2 are measured using a spin echo module with EPI readout, ADC changes significantly as a function of TE, and T2 changes significantly as a function of b-value. Hybrid Multi-dimensional MRI (HM-MRI) imaging measures the change in ADC and T2 as a function of TE and b-value, respectively, and uses these changes as a source of information about the underlying tissue microstructure. As a result, HM-MRI can provide quantitative maps of prostatic tissue composition by exploiting the coupled T2 and ADC values associated with each tissue component.

This pulse sequence was used to acquire images with a set of TEs of (typical values are 47, 75 and 100 msec). At each TE images were acquired with a range of b-values (typical values are 0, 750, and 1500 s/mm$^2$), resulting in a multi-dimensional array of data (if all pair combinations of the foregoing TEs and b-values are acquired, this would result in a 3×3 array) of parameter pair values:

$$\begin{bmatrix} (47\ \text{msec},\ 0\ \text{s/mm}^2) & (47\ \text{msec},\ 750\ \text{s/mm}^2) & (47\ \text{msec},\ 1500\ \text{s/mm}^2) \\ (75\ \text{msec},\ 0\ \text{s/mm}^2) & (75\ \text{msec},\ 750\ \text{s/mm}^2) & (75\ \text{msec},\ 1500\ \text{s/mm}^2) \\ (100\ \text{msec},\ 0\ \text{s/mm}^2) & (100\ \text{msec},\ 750\ \text{s/mm}^2) & (100\ \text{msec},\ 1500\ \text{s/mm}^2) \end{bmatrix}$$

associated with each image voxel. HM-MRI images were acquired in the axial plane and oriented perpendicular to the rectal wall, as guided by sagittal images. Fat saturation was performed using spectrally adiabatic inversion recovery (SPAIR).

The MR signal from prostate tissue is modelled as unmixed pools of water in three tissue components: stroma, epithelium and lumen. Tissue component volumes were calculated on a voxel by voxel basis by fitting Equation 3 (corresponding to Equation 1 for the specific three-tissue-type prostate model):

$$\frac{S}{S_0} =$$

$$V_{stroma}\, exp\left(-\frac{TE}{T_{2_{stroma}}}\right) \exp(-b \times ADC_{stroma}) +$$

$$V_{epithelium}\, exp\left(-\frac{TE}{T_{2_{epithelium}}}\right) \exp\left(-b \times ADC_{epithelium}\right) +$$

$$V_{lumen}\, exp\left(-\frac{TE}{T_{2_{lumen}}}\right) \exp(-b \times ADC_{lumen}) \qquad\qquad (3)$$

where $V_{stroma}$, $V_{epithelium}$, and $V_{lumen}$ are the volume fractions of each compartment, $T_{2stroma}$, $T_{2epithelium}$, and $T_{2lumen}$ are the $T_2$'s for each compartment and $ADC_{stroma}$, $ADC_{epithelium}$ and $ADC_{lumen}$ are the ADC's for each compartment.

In addition, only three tissue components (stroma, epithelium and lumen) are present (e.g. the contribution from blood was not estimated), so that the sum of volume fraction of all three tissue components is one, as expressed in Equation 4 (corresponding to Equation (2) for the specific three-tissue-type prostate model):

$$V_{stroma} + V_{epithelium} + V_{lumen} = 1 \qquad\qquad (4)$$

Anterior Fibromuscular Stroma (AFMS) has dense stromal fibers and is therefore assumed to have lower ADC and $T_2$ value than stroma in the rest of the prostate (see, e.g., Amin M, Khalid A, Tazeen N, Yasoob M. Zonal Anatomy of Prostate. Annals of King Edward Medical University. 2011;16(3); Ward E, Baad M, Peng Y, et al. Multi-parametric MR imaging of the anterior fibromuscular stroma and its differentiation from prostate cancer. Abdom Radiol. 2016:1-9; and McNeal JE. Normal histology of the prostate. Am J Surg Pathol. 1988;12(8):619-33). The lower limits of stromal ADC and $T_2$ were determined based on the ADC's (0.7 $\mu m^2/ms$) and $T_2$'s (40 ms) measured in the AFMS (see, e.g., Niaf E, Lartizien C, Bratan F, et al. Prostate Focal Peripheral Zone Lesions: Characterization at Multiparametric MR Imaging-Influence of a Computer-aided Diagnosis System. Radiology. 2014;271(3):761-9; Baur AD, Maxeiner A, Franiel T, et al. Evaluation of the prostate imaging reporting and data system for the detection of prostate cancer by the results of targeted biopsy of the prostate. Invest Radiol. 2014;49(6):411-20; Hoeks CMA, Vos EK, Bomers JGR, Barentsz JO, Hulsbergen-van de Kaa CA, Scheenen TW. Diffusion-Weighted Magnetic Resonance Imaging in the Prostate Transition Zone: Histopathological Validation Using Magnetic Resonance-Guided Biopsy Specimens. Investigative Radiology. 2013;48(10):693-701; Akin O, Sala E, Moskowitz CS, et al. Transition Zone Prostate Cancers: Features, Detection, Localization, and Staging at Endorectal MR Imaging. Radiology. 2006;239(3):784-92; Shiraishi T, Chikui T, Yoshiura K, Yuasa K. Evaluation of T(2) values and apparent diffusion coefficient of the masseter muscle by clenching. Dentomaxillofacial Radiology. 2011;40(1):35-41; and Langer DL, van der Kwast TH, Evans AJ, et al. Intermixed normal tissue within prostate cancer: effect on MR imaging measurements of apparent diffusion coefficient and T2--sparse versus dense cancers. Radiology. 2008;249(3):900-8).

Cancer is associated with reduced ADC and $T_2$. While cancer is primarily composed of epithelium, 'pure' epithelium will likely have even lower ADC and $T_2$ values than cancer lesions (4). Therefore, the ADC (e.g., 0.7 $\mu m^2/ms$) and $T_2$ (e.g., 70 ms) in high grade values obtained from high grade cancer were used as the upper limit for the epithelium component.

The upper limit for fitting luminal ADC and were set lower than the values of pure water at 37°C. Luminal fluid should have lower ADC and $T_2$ due to the presence of proteins.

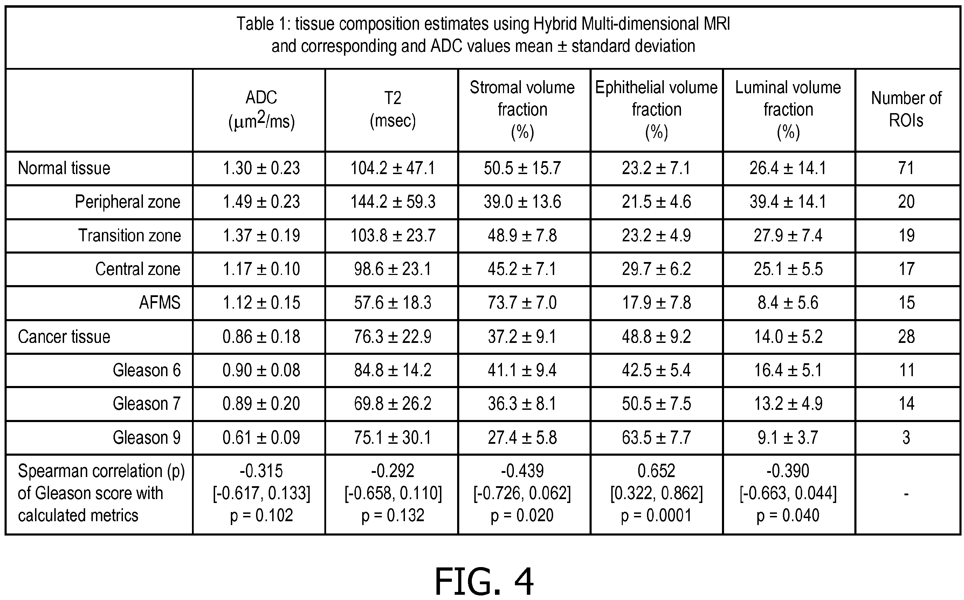

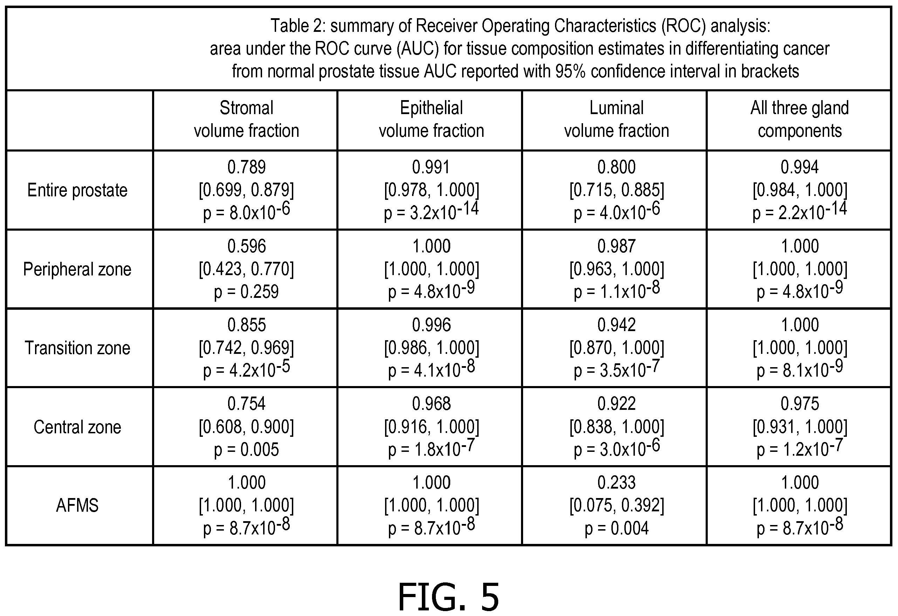

FIGURES 4 and 5 show results from a pilot study. The shaded values show the ability of the computed pTCM values to detect cancer and predict cancer aggressiveness.

The disclosure has been described with reference to the preferred embodiments. Modifications and alterations may occur to others upon reading and understanding the preceding detailed description. It is intended that the disclosure be construed as including all such modifications and alterations insofar as they come within the scope of the appended claims.

## Claims

1.  A non-transitory storage medium storing instructions readable and executable by at least one electronic processor (**20**), wherein the instructions when executed by the at least one electronic processor cause the at least one electronic processor to perform an imaging method (**100**), the imaging method comprising:

obtaining (112) multi-parametric magnetic resonance, MR, imaging data parameterized by a diffusion weighting or perfusion weighting parameter and a magnetization relaxation parameter for a gland of a patient;

determining (114) volume fraction maps of the gland for each of a plurality of gland tissue compartments from the multi-parametric MR imaging data, the gland tissue compartments comprising a stromal compartment, an epithelial compartment, and a lumen compartment; and

controlling (118) a display device **(24)** to display a tissue composition map comprising or generated from the determined volume fraction maps.

2. The non-transitory storage medium of claim 1, wherein the at least one of a diffusion weighting or perfusion parameter includes a diffusion weighting parameter, and the diffusion weighting parameter comprises one of a b-value, a diffusion time, or a diffusion gradient.

3. The non-transitory storage medium of claim 1, wherein the at least one of a diffusion weighting or perfusion parameter value includes a perfusion weighting parameter.

4. The non-transitory storage medium of any one of claims 1-3, wherein the at least one magnetization relaxation parameter comprises one of echo time ($T_E$) or flip angle.

5. The non-transitory storage medium of any one of claims 1-4, wherein the at least one magnetization relaxation parameter value comprises echo time ($T_E$) and the diffusion weighting parameter value comprises a b-value.

6. The non-transitory storage medium of claim 5, wherein:

the multi-parametric magnetic resonance (MR) imaging data includes MR imaging data acquired for at least nine ($T_E$,b-value) parameter value pairs, and

the determining of the volume fraction maps includes solving a system of equations at each voxel for at least the volume fraction of each tissue compartment wherein the system of equations includes, for each ($T_E$,b-value) parameter value pair, an equation relating MR signal of the voxel acquired with the ($T_E$,b-value) parameter value pair to a weighted sum of signal components for each tissue compartment functionally depending on relaxation time $T_2$ and apparent diffusion coefficient (ADC) of the tissue compartment with each signal component weighted by the volume fraction of the tissue compartment; and

wherein the system of equations at each voxel further includes an equation requiring that the sum of the volume fractions equals one.

7. The non-transitory storage medium of claim 6, wherein the system of equations at each voxel is solved for the volume fraction of each tissue compartment and relaxation time $T_2$ and ADC of each tissue compartment.

8. The non-transitory storage medium of claim 5, wherein:

the multi-parametric magnetic resonance (MR) imaging data includes MR imaging data acquired for at least nine ($T_E$,b-value) parameter value pairs, and

the determining of the volume fraction maps includes solving a system of equations at each voxel for at least the volume fraction $V_{Ti}$ of each tissue compartment $T_i \in \{T\}$ where $\{T\}$ denotes the set of the plurality of tissue compartments, wherein the system of equations includes, for each ($T_E$,b-value) parameter value pair, the equation:

$$\frac{S}{S_0} = \sum_{T_i \in \{T\}} V_{T_i} \exp\left(-\frac{T_E}{T_{2T_i}}\right) \exp\left(-(b \times ADC_{T_i})\right)$$

where $S$ is the MR signal at the voxel, $S_0$ is a constant, $T_E$ and $b$ are the echo time $T_E$ and b-value, respectively, of the ($T_E$,b-value) parameter value pair and $T_{2T_i}$ and $ADC_{Ti}$ denote the relaxation time $T_2$ and ADC of the tissue compartment $T_i$; and

wherein the system of equations at each voxel further includes the equation $\Sigma_{Ti \in \{T\}} V_{Ti} = 1$ requiring that the sum of the volume fractions equals one.

9. The non-transitory storage medium of claim 8, wherein the system of equations at each voxel is solved for the volume fraction $V_{Ti}$ of each tissue compartment and for the relaxation time $T_{2Ti}$ and $ADC_{Ti}$ of each tissue compartment.

10. The non-transitory storage medium of any one of claims 1-5, wherein the determining comprises:
solving a system of equations for each voxel to determine the volume fraction values for each tissue compartment of the plurality of tissue compartments where the system of equations for each voxel includes an equation for each value pair of the diffusion weighting or perfusion weighting parameter and the magnetization relaxation parameter in the multi-parametric MR imaging data; and
wherein the system of equations for each voxel further includes an equation requiring that the sum of the volume fractions equals one.

11. The non-transitory storage medium of any one of claims 1-7, wherein the determining further includes:
determining $T_2$ values and apparent diffusion coefficient (ADC) values for each tissue compartment of the plurality of tissue compartments.

12. The non-transitory storage medium of any one of claims 1-11,

wherein the gland is the prostate, and wherein the displayed tissue composition map is a prostatic tissue composition map, pTCM (130); and
wherein the volume fractions for the stromal compartment, the epithelial compartment, and the lumen compartment are measured non-invasively and *in vivo,* and wherein the pTCM (130) is compared with a corresponding map (134) showing confirmed cancer determined from quantitative analysis of whole mount hematoxylin and eosin stained tissue.

13. The non-transitory storage medium of any one of claims 1-12, wherein the display device is controlled to display the tissue composition map comprising at least one of:

display of the volume fraction map for each tissue compartment of the plurality of tissue compartments; or
display of a single composition map fusing the volume fraction maps for the plurality of tissue compartments.

14. The non-transitory storage medium according to claim 1, wherein the gland is the prostate, and wherein the displayed tissue composition map is a prostate tissue composition map, pTCM, and wherein prostate cancer is differentiated from normal tissue in the pTCM based on an increase in volume fraction in the epithelial compartment as compared to normal tissue and a reduction in volume fraction in the luminal compartment as compared to normal tissue.

15. A medical imaging workstation (18) comprising a processor (20) configured to generate a prostatic tissue composition map, pTCM, by transforming a multi-dimensional array of multi-parametric magnetic resonance, (MR), imaging data parameterized by a diffusion weighting or perfusion weighting parameter and a magnetization relaxation parameter for a prostate of a patient using a model to non-invasively evaluate maps of volume fractions, ADC's, and T2's for each of a plurality of prostate tissue compartments;

wherein the processor is configured to obtain the multi-parametric magnetic resonance (MR) imaging data; and
wherein the prostate tissue compartments comprise a stromal compartment, an epithelial compartment, and a lumen compartment.

16. The medical imaging workstation of claim 15, wherein:

the multi-parametric MR imaging data is parameterized by echo time $T_E$ and b-value and comprises MR imaging data acquired for at least nine ($T_E$,b-value) parameter value pairs; and
the pTCM map is generated by solving a system of equations at each voxel for the volume fractions $V_{stroma}$, $V_{epithelium}$, and $V_{lumen}$ of the stromal compartment, the epithelial compartment, and the lumen compartment respectively and for the relaxation times $T_{2stroma}$, $T_{2epithelium}$, and $T_{2lumen}$ of the stromal compartment, the epithelial compartment, and the lumen compartment respectively and for the apparent diffusion coefficients

$ADC_{stroma}$, $ADC_{epithelium}$, and $ADC_{lumen}$ of the stromal compartment, the epithelial compartment, and the lumen compartment respectively wherein the system of equations includes, for each ($T_E$,b-value) parameter value pair, the equation:

$$\frac{S}{S_0} = V_{stroma} \exp\left(-\frac{T_E}{T_{2_{stroma}}}\right) \exp(-(b \times ADC_{stroma}))$$

$$+ V_{epithelium} \exp\left(-\frac{T_E}{T_{2_{epithelium}}}\right) \exp(-(b \times ADC_{epithelium}))$$

$$+ V_{lumen} \exp\left(-\frac{T_E}{T_{2_{lumen}}}\right) \exp(-(b \times ADC_{lumen}))$$

where $S$ is the MR signal at the voxel, $S_0$ is a constant, and $T_E$ and $b$ are the echo time $T_E$ and b-value, respectively, of the ($T_E$,b-value) parameter value pair; and

wherein the system of equations for each voxel further includes an equation requiring that the sum of the volume fractions equals one.

**Patentansprüche**

1.  Ein nicht nur vorübergehendes Speichermedium, auf dem Anweisungen gespeichert werden, die von mindestens einem elektronischen Prozessor (20) lesbar und ausführbar sind, wobei die Anweisungen, wenn sie von mindestens einem elektronischen Prozessor ausgeführt werden, den mindestens einen elektronischen Prozessor veranlassen, eine Bildgebungsmethode (100) durchzuführen, die Bildgebungsmethode umfasst: Erhalten (112) von Magnetresonanz, MR und Bildgebungsdaten mit mehreren Parametern, parametriert durch einen Diffusion oder Perfusionsgewichtungsparameter und einen Magnetisierungs-Relaxationsparameter für eine Drüse eines Patienten; Bestimmen (114) von Volumenfraktionskarten der Drüse für jede von mehreren Drüsen Gewebeabteilungen aus den Mehrparameter-MR-Bildgebungsdaten, die Gewebeabteilungen der Drüsen umfassen eine stromale Abteilung, ein Epithelabteilung und ein Lumenabteilung; und Steuerung (118) eines Anzeigegerätes (24) zur Anzeige einer Gewebezusammensetzungskarte, welche die ermittelten Volumenfraktionsdarstellungen umfasst oder aus ihnen erzeugt wurde.

2.  Nicht nur vorübergehendes Speichermedium nach Anspruch 1, wobei das mindestens eine aus Diffusionsgewichtungs- oder Perfusionsparameters einen Diffusionsgewichtungsparameter umfasst und der Diffusionsgewichtungsparameter eines aus einem b-Wert, einer Diffusionszeit oder einem Diffusionsgradienten umfasst.

3.  Nicht nur vorübergehendes Speichermedium nach Anspruch 1, wobei das mindestens eine aus Diffusionsgewichtungs- oder Perfusionsparameterwert einen Perfusionsgewichtungs-Parameter umfasst.

4.  Nicht nur vorübergehendes Speichermedium nach einem der Ansprüche 1-3, wobei der mindestens eine Magnetisierungs-Relaxationsparameter eines aus Echozeit ($T_E$) oder Flip-Winkel umfasst.

5.  Nicht nur vorübergehendes Speichermedium nach einem der Ansprüche 1-4, wobei der mindestens eine Magnetisierungs-Relaxationsparameterwert Echozeit ($T_E$) umfasst, und der Diffusionsgewichtungparameterwert einen b-Wert umfasst.

6.  Nicht nur vorübergehendes Speichermedium nach Anspruch 5, wobei:
    Die MR-Bildgebungsdaten mit mehreren Parametern MR-Bildgebungsdaten enthalten, die für mindestens neun ($T_E$, b-Wert) Parameterwertpaare erfasst wurden, und die Bestimmung der Volumenfraktionskarten das Lösen eines Gleichungssystems an jedem Voxel für mindestens den Volumenanteil jeder Gewebeabteilung enthält, wobei das Gleichungssystem für jedes Parameterwertpaar ($T_E$,b-Wert) eine Gleichung umfasst, die das MR-Signal des mit dem Parameterwertpaar ($T_E$,b-Wert) akquirierten Voxels mit einer gewichteten Summe von Signalkomponenten für jedes Gewebeabteilung in Abhängigkeit von der Relaxationszeit $T_2$ und scheinbarem Diffusionskoeffizient (ADC) der Gewebeabteilung in Beziehung setzt, wobei jede Signalkomponente durch den Volumenanteil gewichtet wird der Gewebeabteilung; und wobei das Gleichungssystem bei jedem Voxel weiterhin eine Gleichung enthält, die verlangt, dass die Summe der Volumenfraktionen 1 ergibt.

7. Nicht nur vorübergehendes Speichermedium nach Anspruch 6, wobei das Gleichungssystem an jedem Voxel für den Volumenanteil jeder Gewebeabteilung und die Relaxationszeit $T_2$ und ADC jeder Gewebeabteilung gelöst wird.

8. Nicht nur vorübergehendes Speichermedium nach Anspruch 5, wobei:
Die MR-Bildgebungsdaten mit mehreren Parametern MR-Bildgebungsdaten enthalten, die für mindestens neun ($T_E$, b-Wert) Parameterwertpaare erfasst wurden, und die Bestimmung der Volumenfraktionskarten das Lösen eines Gleichungssystems an jedem Voxel für mindestens die Volumenfraktion $V_{Ti}$ jeder Gewebeabteilung $T_i \varepsilon \{T\}$ enthält, wobei $\{T\}$ den Satz der Mehrzahl von Gewebeabteilungen bezeichnet, wobei das Gleichungssystem für jedes ($T_E$, b-Wert) Parameterwertpaar die Gleichung enthält:

$$\frac{S}{S_0} = \sum_{T_i \in \{T\}} V_{T_i} \exp\left(-\frac{T_E}{T_{2T_i}}\right) \exp\left(-(b \times ADC_{T_i})\right)$$

wobei $S$ das MR-Signal am Voxel, $S_0$ eine Konstante, $T_E$ und $b$ die Echozeit $T_E$ bzw. b-Wert des Parameterwertpaares ($T_E$, b-Wert) und $T_{2Ti}$ und $ADC_{Ti}$ die Relaxationszeit $T_2$ und ADC der Gewebeabteilung $T_i$ bezeichnen, und
wobei das Gleichungssystem bei jedem Voxel weiterhin die Gleichung $\Sigma_{Ti \in \{T\}} V_{Ti} = 1$ enthält, die erfordert, dass die Summe der Volumenanteile gleich eins ist.

9. Nicht nur vorübergehendes Speichermedium nach Anspruch 8, wobei das Gleichungssystem an jedem Voxel für die Volumenfraktion $V_{Ti}$ jeder Gewebeabteilung und für die Relaxationszeit $T_{2Ti}$ und $ADC_{Ti}$ jeder Gewebeabteilung gelöst wird.

10. Nicht nur vorübergehendes Speichermedium nach einem der Ansprüche 1-5, wobei die Bestimmung umfasst: Lösen eines Gleichungssystems für jedes Voxel, um die Volumenfraktionswerte für jede Gewebeabteilung der Mehrzahl von Gewebeabteilungen zu bestimmen, wobei das Gleichungssystem für jedes Voxel eine Gleichung für jedes Wertepaar des Diffusionsgewichtungs- oder Perfusionsgewichtungsparameters und der Magnetisierung und die Relaxationsparameter in den Mehrparamete-MR-Bildgebungsdaten enthält; und wobei das Gleichungssystem für jedes Voxel weiterhin eine Gleichung enthält, die verlangt, dass die Summe der Volumenfraktionen 1 ergibt.

11. Nicht nur vorübergehendes Speichermedium nach einem der Ansprüche 1-7, wobei die Bestimmung des Weiteren umfasst:
Bestimmung der $T_2$-Werte und der scheinbaren Diffusionskoeffizient-Werte (ADC) für jede Gewebeabteilung aus mehreren Gewebeabteilungen.

12. Nicht nur vorübergehendes Speichermedium nach einem der Ansprüche 1-11, wobei die Drüse die Prostata ist und wobei die angezeigte Gewebezusammensetzungskarte eine Prostata-Gewebezusammensetzungskarte pTCM (130) ist; und wobei die Volumenfraktionen für die Stromalabteilung, die Epithelabteilung und die Lumenabteilung nicht-invasiv und *in vivo* gemessen wird, wobei das pTCM (130) mit einer entsprechenden Karte (134) verglichen wird, die bestätigten Krebs zeigt, der aus quantitativer Analyse von Ganzmonthämatoxylin und Eosin-gefärbtem Gewebe bestimmt wurde.

13. Nicht nur vorübergehendes Speichermedium nach einem der Ansprüche 1-12, wobei die Anzeigevorrichtung gesteuert ist, um die Gewebezusammensetzungskarte anzuzeigen, die mindestens einen der folgenden Elemente umfasst:

Anzeige der Volumenfraktion für jede Gewebeabteilung der Vielzahl von Gewebeabteilungen; oder
Anzeige einer einzelnen Kompositionskarte, in der die Volumenfraktionskarten für die Vielzahl von Gewebeabteilungen zusammengeführt werden.

14. Nicht nur vorübergehendes Speichermedium nach Anspruch 1, wobei die Drüse die Prostata ist und wobei die dargestellte Gewebezusammensetzungskarte eine Prostatagewebe-Zusammensetzungskarte, pTCM, ist, und wobei ein Prostatakarzinom von normalem Gewebe im pTCM aufgrund einer Erhöhung der Volumenfraktion in der Epithelabteilung im Vergleich zu normalem Gewebe und einer Verringerung der Volumenfraktion in der Luminalabteilung im Vergleich zu normalem Gewebe unterschieden wird.

15. Workstation für die medizinische Bildgebung (18), die einen Prozessor (20) umfasst, der konfiguriert ist, um eine Karte für die Zusammensetzung des Prostatagewebes, pTCM, zu erzeugen, und zwar durch Umwandlung eines mehrdimensionalen Arrays (MR) der Bildgebung data, parametriert durch einen Diffusionsgewichtungs- oder Perfusionsgewichtungsparameter und einen Magnetisierungs-Relaxationsparameter für eine Prostata eines Patienten, wobei ein Modell zur nichtinvasiven Auswertung von Karten von Volumenfraktionen, ADC, und T2 für jedes der verschiedenen Prostata-Gewebekompartimente verwendet wird;

wobei der Prozessor so konfiguriert ist, dass er die (MR)-Bildgebungsdaten mit mehreren Parametern erhält; und
wobei die Prostata-Gewebekompartimente ein Stromakompartiment, ein Epithelkompartiment und ein Lumenkompartiment umfassen.

16. Workstation für die medizinische Bildgebung nach Anspruch 15, wobei:

Die MR-Bildgebungsdaten mit mehreren Parametern werden durch die Echozeit $T_E$ und den b-Wert parametriert und umfassen MR-Bildgebungsdaten, die für mindestens neun Parameterwertpaare ($T_E$,b-Wert) erfasst wurden; und
die pTCM-Karte wird generiert, indem an jedem Voxel ein Gleichungssystem für die Volumenfraktionen $V_{stroma}$, $V_{epithel}$ und $V_{lumen}$ des Stromakompartiments, des Epithelkompartiments und des Lumenkompartiments gelöst wird, ebenso auch für die Relaxationszeiten $T_{2stroma}$, $T_{2epithel}$ und $T_{2lumen}$ des Stromakompartiments, des Epithelkompartiments und des Lumenkompartiments sowie für die scheinbaren Diffusionskoeffizienten $ADC_{stroma}$, $ADC_{epithelium}$, und $ADC_{lumen}$ des Stromakompartiments, des Epithelkompartiments und des Lumenkompartiments,
wobei das Gleichungssystem für jedes Parameterwertpaar ($T_E$, b-Wert) die Gleichung umfasst:

$$\frac{S}{S_0} = V_{stroma} \exp\left(-\frac{T_E}{T_{2_{stroma}}}\right) \exp\left(-(bx\,ADC_{stroma})\right)$$

$$+V_{epithel} \exp\left(-\frac{T_E}{T_{2_{epithel}}}\right) \exp\left(-(bx\,ADC_{epithel})\right)$$

$$+V_{lumen} \exp\left(-\frac{T_E}{T_{2_{lumen}}}\right) \exp\left(-(bx\,ADC_{lumen})\right)$$

wobei S das MR-Signal am voxel ist, $S_0$ eine Konstante und $T_E$ und b die Echozeit $T_E$ bzw. Der b-Wert des Parameterwertpaares ($T_E$, b-Wert) sind; und
wobei das Gleichungssystem für jedes voxel eine Gleichung enthält, die verlangt, dass die Summe der Volumenfraktionen 1 ergibt.

## Revendications

1. Support de stockage non transitoire stockant des instructions lisibles et exécutables par au moins un processeur électronique (20), dans lequel les instructions, lorsqu'elles sont exécutées par au moins un processeur électronique, amènent ce dernier à exécuter une méthode d'imagerie (100), la méthode d'imagerie comprenant:

obtenir (112) des données IRM par résonance magnétique multiparamétriques paramétrées par un paramètre de pondération de la diffusion ou de la perfusion et un paramètre de relaxation de l'aimantation pour une glande d'un patient; déterminer (114) des cartes de fraction volumique de la glande pour chacun des compartiments de tissu de la glande à partir des données d'imagerie IRM multiparamétrique, les compartiments de tissu de la glande comprenant un compartiment stromal, un compartiment épithélial et un compartiment de lumière; et commander (118) un dispositif d'affichage (24) pour afficher une carte de composition tissulaire comprenant ou générée à partir des cartes de fraction volumique déterminées.

**2.** Support de stockage non transitoire selon la revendication 1, dans lequel au moins l'un des paramètres de pondération de la diffusion ou de la perfusion comprend un paramètre de pondération de la diffusion, et le paramètre de pondération de la diffusion comprend l'une des valeurs b, un temps de diffusion ou un gradient de diffusion.

**3.** Support de stockage non transitoire selon la revendication 1, dans lequel au moins l'une des valeurs d'un paramètre de pondération de la diffusion ou de la perfusion comprend un paramètre de pondération de la perfusion.

**4.** Support de stockage non transitoire selon l'une des revendications 1 à 3, dans lequel l'au moins un paramètre de relaxation de l'aimantation comprend le temps d'écho ($T_E$) ou l'angle de bascule.

**5.** Support de stockage non transitoire selon l'une des revendications 1 à 4, dans lequel la valeur du paramètre de relaxation de l'aimantation au moins comprend le temps d'écho ($T_E$) et la valeur du paramètre de pondération de la diffusion comprend une valeur b.

**6.** Le support de stockage non transitoire de la revendication 5, dans lequel:

les données d'imagerie par résonance magnétique (IRM) multiparamétriques comprennent des données d'imagerie IRM acquises pour au moins neuf paires de valeurs de paramètres ($T_E$, valeur b), et la détermination des cartes de fraction volumique comprend la résolution d'un système d'équations à chaque voxel pour au moins la fraction volumique de chaque compartiment tissulaire, le système d'équations comprenant, pour chaque paire de valeurs de paramètres ($T_E$, valeur b), une équation reliant le signal IRM du voxel acquis avec la paire de valeurs de paramètres ($T_E$, valeur b) à une somme pondérée de composantes de signal pour chaque compartiment tissulaire dépendant fonctionnellement du temps de relaxation $T_2$ et du coefficient de diffusion apparent (ADC) du compartiment tissulaire, chaque composante de signal étant pondérée par la fraction volumique du compartiment tissulaire; et
où le système d'équations à chaque voxel comprend en outre une équation exigeant que la somme des fractions volumiques soit égale à un.

**7.** Support de stockage non transitoire selon la revendication 6, dans lequel le système d'équations à chaque voxel est résolu pour la fraction volumique de chaque compartiment de tissu et le temps de relaxation $T_2$ et ADC de chaque compartiment de tissu.

**8.** Support de stockage non transitoire selon la revendication 5, dans lequel:

les données d'imagerie par résonance magnétique (IRM) multiparamétriques comprennent des données d'imagerie IRM acquises pour au moins neuf paires de valeurs de paramètres ($T_E$, valeur b), et
la détermination des cartes de fraction volumique comprend la résolution d'un système d'équations à chaque voxel pour au moins la fraction volumique $V_{Ti}$ de chaque compartiment tissulaire $T_i \varepsilon \{T\}$ où $\{T\}$ désigne l'ensemble de la pluralité de compartiments tissulaires, le système d'équations comprenant, pour chaque paire de valeurs de paramètres ($T_E$, valeur b), l'équation:

$$\frac{S}{S_0} = \sum_{T_i \in \{T\}} V_{T_i} \exp\left(-\frac{T_E}{T_{2T_i}}\right) \exp\left(-(b \times ADC_{T_i})\right)$$

où S est le signal IRM au niveau du voxel, $S_0$ est une constante, $T_E$ e b sont respectivement le temps d'écho $T_E$ et la valeur b de la paire de paramètres ($T_E$,b-value) et $T_{2Ti}$ et $ADC_{Ti}$ désignent le temps de relaxation $T_2$ et ADC du compartiment tissulaire $T_i$; et dans lequel le système d'équations à chaque voxel comprend en outre l'équation $\Sigma_{Ti \in \{T\}} V_{Ti} = 1$ exigeant que la somme des fractions volumiques soit égale à un.

**9.** Support de stockage non transitoire selon la revendication 8, dans lequel le système d'équations à chaque voxel est résolu pour la fraction volumique $V_{Ti}$ de chaque compartiment tissulaire et pour le temps de relaxation $T_{2Ti}$ et $ADC_{Ti}$ de chaque compartiment tissulaire.

**10.** Support de stockage non transitoire selon l'une des revendications 1 à 5, dans lequel la détermination comprend:

résoudre un système d'équations pour chaque voxel afin de déterminer les valeurs de fraction volumique pour chaque compartiment tissulaire de la pluralité de compartiments tissulaires, le système d'équations pour chaque voxel comprenant une équation pour chaque paire de valeurs du paramètre de pondération de la diffusion ou de la perfusion et du paramètre de relaxation de l'aimantation dans les données d'imagerie IRM multiparamétriques; et

où le système d'équations pour chaque voxel comprend en outre une équation exigeant que la somme des fractions volumiques soit égale à un.

**11.** Support de stockage non transitoire selon l'une des revendications 1 à 7, dans lequel la détermination comprend en outre:

déterminer les valeurs $T_2$ et les valeurs du coefficient de diffusion apparent (ADC) pour chaque compartiment tissulaire de la pluralité de compartiments tissulaires.

**12.** Support de stockage non transitoire selon l'une des revendications 1 à 11, dans lequel la glande est la prostate, et dans lequel la carte de composition tissulaire affichée est une carte de composition tissulaire prostatique, pTCM (130); et

dans lequel les fractions volumiques pour le compartiment stromal, le compartiment épithélial et le compartiment de lumière sont mesurées de manière non invasive et *in vivo*, et dans laquelle la carte pTCM (130) est comparée à une carte correspondante (134) montrant un cancer confirmé déterminé à partir d'une analyse quantitative de tissus entiers colorés à l'hématoxyline et à l'éosine.

**13.** Support de stockage non transitoire selon l'une des revendications 1 à 12, dans lequel le dispositif d'affichage est commandé pour afficher la carte de composition tissulaire comprenant au moins l'un des éléments suivants:

l'affichage de la carte de fraction volumique pour chaque compartiment tissulaire de la pluralité de compartiments tissulaires; ou

l'affichage d'une carte de composition unique fusionnant les cartes de fraction volumique pour la pluralité de compartiments tissulaires.

**14.** Support de stockage non transitoire selon la revendication 1, dans lequel la glande est la prostate, et dans lequel la carte de composition tissulaire affichée est une carte de composition tissulaire de la prostate, pTCM, et dans laquelle le cancer de la prostate est différencié du tissu normal dans le pTCM sur la base d'une augmentation de la fraction volumique dans le compartiment épithélial par rapport au tissu normal et d'une réduction de la fraction volumique dans le compartiment de lumière par rapport au tissu normal.

**15.** Station de travail d'imagerie médicale (18) comprenant un processeur (20) configuré pour générer une carte de composition tissulaire prostatique, pTCM, par transformation d'un réseau multidimensionnel par résonance magnétique multiparamétrique (MR), données d'imagerie paramétrées par un paramètre de pondération de diffusion ou de perfusion et un paramètre de relaxation de magnétisation pour la prostate d'un patient à l'aide d'un modèle pour évaluer de manière non invasive des cartes de fractions volumiques, ADC, et T2 pour chacun des compartiments de tissu prostatique;

le processeur étant configuré pour obtenir les données d'imagerie par résonance magnétique (MR) multiparamétriques; et

où les compartiments de tissu prostatique comprennent un compartiment stromal, un compartiment épithélial et un compartiment de lumière.

**16.** Station de travail d'imagerie médicale selon la revendication 15, dans laquelle:

les données d'imagerie par résonance magnétique multiparamétriques sont paramétrées par le temps d'écho $T_E$ et la valeur b et comprennent des données d'imagerie par résonance magnétique acquises pour au moins neuf paires de valeurs de paramètres ($T_E$, valeur b); et

la cartographie pTCM est générée en résolvant un système d'équations au niveau de chaque voxel pour les fractions volumiques $V_{stroma}$, $V_{épithélium}$ et $V_{lumen}$ du compartiment stromal, du compartiment épithélial, et le compartiment de lumière respectivement et pour les temps de relaxation $T_{2stroma}$, $T_{2épithélium}$ et $T_{2lumen}$ du compartiment stromal, du compartiment épithélial et du compartiment de lumière respectivement et pour les coefficients de diffusion apparente $ADC_{stroma}$, $ADC_{épithélium}$, et $ADC_{lumen}$ du compartiment stromal, du compartiment épithélial et du compartiment lumen respectivement, dans lesquels le système d'équations comprend, pour chaque paire de valeurs de paramètre ($T_E$, valeur b), l'équation:

$$\frac{S}{S_0} = V_{stroma} \exp\left(-\frac{T_E}{T_{2_{stroma}}}\right) \exp\left(-(bx\ ADC_{stroma})\right)$$

$$+ V_{\text{épithélium}} \exp\left(-\frac{T_E}{T_{2_{\text{épithélium}}}}\right) \exp\left(-(bx\ ADC_{\text{épithélium}})\right)$$

$$+ V_{lumen} \exp\left(-\frac{T_E}{T_{2_{lumen}}}\right) \exp\left(-(bx\ ADC_{lumen})\right)$$

où S est le signal MR au voxel, $S_0$ est une constante, et $T_E$ et b sont respectivement le temps d'écho $T_E$ et la valeur b de la paire de valeurs du paramètre ($T_E$, valeur b); et

dans lequel le système d'équations pour chaque voxel comprend en outre une équation exigeant que la somme des fractions volumiques soit égale à un.

FIG. 1

100 {

Obtain multi-parametric MR imaging data parameterized by
a diffusion weighting or perfusion weighting parameter and
a magnetization relaxation parameter for a Region Of
Interest (ROI) of a patient
<u>112</u>

Determine volume fraction maps of the ROI for each of a plurality
of tissue types from the multi-parameteric MR imaging data
<u>114</u>

106 {

Generate individual renderings for the volume fraction
map of each tissue type; or, a single composite rendering
fusing the volume fraction maps
<u>116</u>

Control the display device to display a tissue composition map
comprising or generated from the determined volume
fraction maps
<u>118</u>

# FIG. 2

FIG. 3

| | ADC ($\mu m^2$/ms) | T2 (msec) | Stromal volume fraction (%) | Ephithelial volume fraction (%) | Luminal volume fraction (%) | Number of ROIs |
|---|---|---|---|---|---|---|
| Table 1: tissue composition estimates using Hybrid Multi-dimensional MRI and corresponding and ADC values mean ± standard deviation | | | | | | |
| Normal tissue | 1.30 ± 0.23 | 104.2 ± 47.1 | 50.5 ± 15.7 | 23.2 ± 7.1 | 26.4 ± 14.1 | 71 |
| Peripheral zone | 1.49 ± 0.23 | 144.2 ± 59.3 | 39.0 ± 13.6 | 21.5 ± 4.6 | 39.4 ± 14.1 | 20 |
| Transition zone | 1.37 ± 0.19 | 103.8 ± 23.7 | 48.9 ± 7.8 | 23.2 ± 4.9 | 27.9 ± 7.4 | 19 |
| Central zone | 1.17 ± 0.10 | 98.6 ± 23.1 | 45.2 ± 7.1 | 29.7 ± 6.2 | 25.1 ± 5.5 | 17 |
| AFMS | 1.12 ± 0.15 | 57.6 ± 18.3 | 73.7 ± 7.0 | 17.9 ± 7.8 | 8.4 ± 5.6 | 15 |
| Cancer tissue | 0.86 ± 0.18 | 76.3 ± 22.9 | 37.2 ± 9.1 | 48.8 ± 9.2 | 14.0 ± 5.2 | 28 |
| Gleason 6 | 0.90 ± 0.08 | 84.8 ± 14.2 | 41.1 ± 9.4 | 42.5 ± 5.4 | 16.4 ± 5.1 | 11 |
| Gleason 7 | 0.89 ± 0.20 | 69.8 ± 26.2 | 36.3 ± 8.1 | 50.5 ± 7.5 | 13.2 ± 4.9 | 14 |
| Gleason 9 | 0.61 ± 0.09 | 75.1 ± 30.1 | 27.4 ± 5.8 | 63.5 ± 7.7 | 9.1 ± 3.7 | 3 |
| Spearman correlation (p) of Gleason score with calculated metrics | -0.315 [-0.617, 0.133] p = 0.102 | -0.292 [-0.658, 0.110] p = 0.132 | -0.439 [-0.726, 0.062] p = 0.020 | 0.652 [0.322, 0.862] p = 0.0001 | -0.390 [-0.663, 0.044] p = 0.040 | - |

## FIG. 4

| | Stromal volume fraction | Epithelial volume fraction | Luminal volume fraction | All three gland components |
|---|---|---|---|---|
| Entire prostate | 0.789 [0.699, 0.879] $p = 8.0 \times 10^{-6}$ | 0.991 [0.978, 1.000] $p = 3.2 \times 10^{-14}$ | 0.800 [0.715, 0.885] $p = 4.0 \times 10^{-6}$ | 0.994 [0.984, 1.000] $p = 2.2 \times 10^{-14}$ |
| Peripheral zone | 0.596 [0.423, 0.770] $p = 0.259$ | 1.000 [1.000, 1.000] $p = 4.8 \times 10^{-9}$ | 0.987 [0.963, 1.000] $p = 1.1 \times 10^{-8}$ | 1.000 [1.000, 1.000] $p = 4.8 \times 10^{-9}$ |
| Transition zone | 0.855 [0.742, 0.969] $p = 4.2 \times 10^{-5}$ | 0.996 [0.986, 1.000] $p = 4.1 \times 10^{-8}$ | 0.942 [0.870, 1.000] $p = 3.5 \times 10^{-7}$ | 1.000 [1.000, 1.000] $p = 8.1 \times 10^{-9}$ |
| Central zone | 0.754 [0.608, 0.900] $p = 0.005$ | 0.968 [0.916, 1.000] $p = 1.8 \times 10^{-7}$ | 0.922 [0.838, 1.000] $p = 3.0 \times 10^{-6}$ | 0.975 [0.931, 1.000] $p = 1.2 \times 10^{-7}$ |
| AFMS | 1.000 [1.000, 1.000] $p = 8.7 \times 10^{-8}$ | 1.000 [1.000, 1.000] $p = 8.7 \times 10^{-8}$ | 0.233 [0.075, 0.392] $p = 0.004$ | 1.000 [1.000, 1.000] $p = 8.7 \times 10^{-8}$ |

Table 2: summary of Receiver Operating Characteristics (ROC) analysis: area under the ROC curve (AUC) for tissue composition estimates in differentiating cancer from normal prostate tissue AUC reported with 95% confidence interval in brackets

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SIEGEL R ; NAISHADHAM D ; JEMAL A.** Cancer statistics, 2012. *CA: A Cancer Journal for Clinicians,* 2012, vol. 62 (1), 10-29
- **JOHNSON LM ; CHOYKE PL ; FIGG WD ; TURKBEY B.** The Role of MRI in Prostate Cancer Active Surveillance. *BioMed Research International.,* 2014, vol. 2014, 6
- **CHATTERJEE A ; WATSON G ; MYINT E ; SVED P ; MCENTEE M ; BOUME R.** Changes in Epithelium, Stroma, and Lumen Space Correlate More Strongly with Gleason Pattern and Are Stronger Predictors of Prostate ADC Changes than Cellularity Metrics. *Radiology.,* 2015, vol. 277 (3), 751-62
- **LANGER DL ; VAN DER KWAST TH ; EVANS AJ et al.** Prostate tissue composition and MR measurements: investigating the relationships between ADC, T2, K(trans), v(e), and corresponding histologic features. *Radiology.,* 2010, vol. 255 (2), 485-94
- **KOBUS T ; LAAK JAWMVD ; MAAS MC et al.** Contribution of Histopathologic Tissue Composition to Quantitative MR Spectroscopy and Diffusion-weighted Imaging of the Prostate. *Radiology.,* 2015, vol. 278 (3), 801-11
- **ZHAO M ; MYINT E ; WATSON G ; BOURNE R.** Comparison of conventional histology and diffusion weighted microimaging for estimation of epithelial, stromal, and acinar volumes in prostate tissue. *Proceedings of the 21st Annual Meeting of ISMRM.,* 2013, 3090
- **BOURNE RM ; KURNIAWAN N ; COWIN G et al.** Microscopic diffusivity compartmentation in formalin-fixed prostate tissue. *Magn Reson Med.,* 2012, vol. 68 (2), 614-20
- **BOURNE R ; KURNIAWAN N ; COWIN G ; SVED P ; WATSON G.** 16 T diffusion microimaging of fixed prostate tissue: preliminary findings. *Magn Reson Med.,* 2011, vol. 66 (1), 244-7
- **BOURNE R.** Magnetic resonance microscopy of prostate tissue: How basic science can inform clinical imaging development. *Journal of Medical Radiation Sciences.,* 2013, vol. 60 (1), 5-10
- **KITZING YX ; PRANDO A ; VAROL C ; KARCZMAR GS ; MACLEAN F ; OTO A.** Benign Conditions That Mimic Prostate Carcinoma: MR Imaging Features with Histopathologic Correlation. *RadioGraphics.,* 2016, vol. 36 (1), 162-75

- **ROSENKRANTZ AB ; TANEJA SS.** Radiologist, Be Aware: Ten Pitfalls That Confound the Interpretation of Multiparametric Prostate MRI. *American Journal of Roentgenology.,* 2013, vol. 202 (1), 109-20
- **PANAGIOTAKI E ; CHAN RW ; DIKAIOS N et al.** Microstructural Characterization of Normal and Malignant Human Prostate Tissue With Vascular, Extracellular, and Restricted Diffusion for Cytometry in Tumours Magnetic Resonance Imaging. *Investigative radiology.,* 2015, vol. 50 (4), 218-27
- **SELNÆS KM ; VETTUKATTIL R ; BERTILSSON H et al.** Tissue Microstructure Is Linked to MRI Parameters and Metabolite Levels in Prostate Cancer. *Frontiers in Oncology.,* 2016, vol. 6, 146
- **SABOURI S ; FAZLI L ; CHANG SD et al.** MR measurement of luminal water in prostate gland: Quantitative correlation between MRI and histology. *Journal of Magnetic Resonance Imaging.,* 2017
- **SABOURI S ; CHANG SD ; SAVDIE R et al.** Luminal Water Imaging: A New MR Imaging T2 Mapping Technique for Prostate Cancer Diagnosis. *Radiology.,* 2017, vol. 0 (0), 161687
- **JEROME, N. P. et al.** Extended T2-IVIM model for correction of TE dependence of pseudo-diffusion volume fraction in clinical diffusion-weighted magnetic resonance imaging. *Phys. Med. Biol.,* 2016, vol. 61, N667-N680
- **MELBOURNE, A. et al.** Multi-modal Measurement of the Myelin-to-Axon Diameter g-ratio in Preterm-born Neonates and Adult Controls. *SAT 2015 18th International Conference; Lecture Notes in Computer Science,* September 2014, 268-275
- **RYDHÖG, A. S. et al.** Multidimensional Diffusion and Relaxation Data Acquisition for Improved Intravoxel Incoherent Motion Analysis. *Proceedings of the International Society for Magnetic Resonance in Medicine,* April 2016, vol. 24, 1897
- **CHATTERJEE, A. ; BOURNE, R.M. ; KARCZMAR, G.S ; OTO, A. et al.** Diagnosis of Prostate Cancer with Noninvasive Estimation of Prostate Tissue Composition by using Hybrid Multidimensional MR imaging: A Feasibility Study. *Radiology,* 2018
- **STORÅS TH ; GJESDAL K-I ; GADMAR ØB ; GEITUNG JT ; KLØW N-E.** Prostate magnetic resonance imaging: Multiexponential T2 decay in prostate tissue. *Journal of Magnetic Resonance Imaging.,* 2008, vol. 28 (5), 1166-72

- **KJAER L ; THOMSEN C ; IVERSEN P ; HENRIK-SEN O.** In vivo estimation of relaxation processes in benign hyperplasia and carcinoma of the prostate gland by magnetic resonance imaging. *Magnetic resonance imaging.,* 1987, vol. 5 (1), 23-30
- **BOURNE R ; PANAGIOTAKI E.** Limitations and Prospects for Diffusion-Weighted MRI of the Prostate. *Diagnostics.,* 2016, vol. 6 (2), 21
- **WANGE, S. ; OTO, A. ; KARCZMAR, G.S.** Hybrid Multidimentional T2 and diffusion-weighted MRI for prostate cancer detection. *J Magnetic Resonance Imaging,* 2014
- **AMIN M ; KHALID A ; TAZEEN N ; YASOOB M.** Zonal Anatomy of Prostate. *Annals of King Edward Medical University.,* 2011, vol. 16 (3
- **WARD E ; BAAD M ; PENG Y et al.** Multi-parametric MR imaging of the anterior fibromuscular stroma and its differentiation from prostate cancer. *Abdom Radiol.,* 2016, 1-9
- **MCNEAL JE.** Normal histology of the prostate. *Am J Surg Pathol.,* 1988, vol. 12 (8), 619-33
- **NIAF E ; LARTIZIEN C ; BRATAN F et al.** Prostate Focal Peripheral Zone Lesions: Characterization at Multiparametric MR Imaging-Influence of a Computer-aided Diagnosis System. *Radiology.,* 2014, vol. 271 (3), 761-9
- **BAUR AD ; MAXEINER A ; FRANIEL T et al.** Evaluation of the prostate imaging reporting and data system for the detection of prostate cancer by the results of targeted biopsy of the prostate. *Invest Radiol.,* 2014, vol. 49 (6), 411-20
- **VOS EK ; BOMERS JGR ; BARENTSZ JO ; HULSBERGEN-VAN DE KAA CA ; SCHEENEN TW.** Diffusion-Weighted Magnetic Resonance Imaging in the Prostate Transition Zone: Histopathological Validation Using Magnetic Resonance-Guided Biopsy Specimens. *Investigative Radiology.,* 2013, vol. 48 (10), 693-701
- **AKIN O ; SALA E ; MOSKOWITZ CS et al.** Transition Zone Prostate Cancers: Features, Detection, Localization, and Staging at Endorectal MR Imaging. *Radiology,* 2006, vol. 239 (3), 784-92
- **SHIRAISHI T ; CHIKUI T ; YOSHIURA K ; YUASA K.** Evaluation of T(2) values and apparent diffusion coefficient of the masseter muscle by clenching. *Dentomaxillofacial Radiology.,* 2011, vol. 40 (1), 35-41
- **LANGER DL ; VAN DER KWAST TH ; EVANS AJ et al.** Intermixed normal tissue within prostate cancer: effect on MR imaging measurements of apparent diffusion coefficient and T2--sparse versus dense cancers. *Radiology.,* 2008, vol. 249 (3), 900-8